# EUROPEAN PATENT SPECIFICATION

(11) **EP 3 407 874 B1**
(45) Date of publication and mention of the grant of the patent: **22.05.2024**
(21) Application number: 17701495.8
(22) Date of filing: 25.01.2017
(51) Int. Cl.: A61K 9/20, A61K 31/506

(54) **FAST DISPERSIBLE PHARMACEUTICAL COMPOSITION COMPRISING TYROSINE-KINASE INHIBITOR**
SCHNELL DISPERGIERBARE PHARMAZEUTISCHE ZUSAMMENSETZUNG MIT TYROSIN-KINASE-INHIBITOREN
COMPOSITION PHARMACEUTIQUE À DISPERSION RAPIDE COMPRENANT UN INHIBITEUR DE LA TYROSINE KINASE

(30) Priority: 25.01.2016 SI 201600024
(43) Date of publication of application: 05.12.2018
(73) Proprietor: KRKA, d.d., Novo mesto, 8000 Novo mesto (SI)
(72) Inventor: KORASA, Klemen, 8501 NOVO MESTO (SI); KUKEC, Simon, 8501 NOVO MESTO (SI); KROSELJ, Vesna, 8501 NOVO MESTO (SI); VRECER, Franc, 8501 NOVO MESTO (SI); SKRABANJA, Vida, 8501 NOVO MESTO (SI); GERMAN ILIC, Tamara, 8501 NOVO MESTO (SI)
(74) Representative: Hoefer & Partner Patentanwälte mbB
(86) International application number: PCT/EP2017/051561
(87) International publication number: WO 2017/129624

(56) References cited:
- EP-A1- 2 801 349
- WO-A1-2011/160798
- CN-A- 101 401 795
- CN-A- 102 302 464
- CN-A- 103 751 140
- KR-A- 20140 065 862
- VADERA ET AL: "Stability-indicating HPTLC determination of imatinib mesylate in bulk drug and pharmaceutical dosage form", JOURNAL OF PHARMACEUTICAL AND BIOMEDICAL ANALYSIS, NEW YORK, NY, US, vol. 43, no. 2, 23 December 2006 (2006-12-23), pages 722-726, XP005735145, ISSN: 0731-7085, DOI: 10.1016/J.JPBA.2006.07.022
- Robert G. Strickley ET AL: "Pediatric drugs-a review of commercially available oral formulations", Journal of Pharmaceutical Sciences, vol. 97, no. 5, 1 May 2008 (2008-05-01), pages 1731-1774, XP055121290, ISSN: 0022-3549, DOI: 10.1002/jps.21101

## Description

### FIELD OF THE INVENTION

The present invention relates to a stable pharmaceutical composition comprising tyrosine-kinase inhibitor, which is monomesylate salt of imatinib, together with pharmaceutically acceptable excipients intended to be dispersed in water and/or other aqueous liquids before oral administration. The invention also refers to the process for the preparation of said pharmaceutical preparations with an improved stability and purity, characterized by fast disintegration in water or other aqueous liquids such as fruit juices where fast disintegration means that said pharmaceutical composition disintegrates in less than 3 minutes using purified water at 25°C. Furthermore, the present invention pertains to the use of a dispersible pharmaceutical composition in the form of tablet(s) for preparing an oral administration dispersion in water and/or other aqueous liquids before oral administration.

### BACKGROUND OF THE INVENTION

Imatinib is the generic name for N-(4-methyl-3-((4-(pyridin-3-yl)pyrimidin-2-yl)amino)phenyl)-4-((4-methylpiperazin-1-yl)methyl)benzamide of the following formula (I):

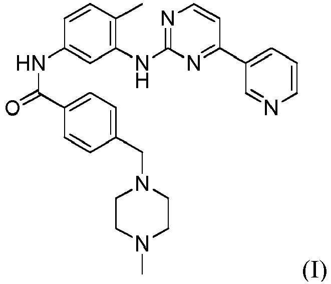

The preparation of imatinib and the use thereof, especially in the chemotherapy of tumors, was first disclosed in EP0564409. The compound also displays polymorphic structures which are known from WO9903854, WO2007023182, WO20050779333, WO2006054314, WO20040106326, WO2007136510, US2009181977, WO2009147626. Various different acid addition salts of imatinib are described in WO2004074502, WO2005075454 and WO2009065910.

A publication of Robert G. Strickley et al. in the "Journal oƒ Pharmaceutical Sciences", vol. 97, no. 5, May 2008, pages 1731 to 1774 ("*Pediatric Drugs* - *A Review oƒ Commercially Available Oral Formulations"*) indicates that pediatric oral formulations can be quite scientifically challenging to develop. Table 3 thereof shows a *"Selected Listing oƒ Commercially Available Pediatric Oral Formulations-Manipulation Required".*

The term »imatinib« used herein refers to above named compound (I) as well as pharmaceutically acceptable salts, enantiomers, racemates, solvates, hydrates, amorphous and crystal forms or combinations thereof.

Imatinib is known as a member of agents that act by inhibiting particular tyrosine kinase enzymes and is indicated for the treatment of chronic myeloid leukemia (CML), gastrointestinal stromal tumors (GISTs) and a number of other malignancies. Imatinib may also have a role in the treatment of pulmonary hypertension. The drug is currently being marketed as its monomethanesulfonate salt under the trade names Gleevec^{®} and Glivec^{®} in oral dosage forms such as hard gelatin capsules (100 mg) and coated tablets (100 mg and 400 mg).

The substantial antileukemic activity of imatinib in adult CML trials prompted investigators to evaluate this agent in children with recurrent or refractory Ph+ leukemias. Because of limited availability of pediatric drug formulations, commonly the adult-strength drug products are reformulated to meet the suitable dosing for pediatric patients. Pharmacists and pediatricians are especially faced with the problem of modifying an oral dose form for adult use into a suitable form for pediatric patients when anticancer drug is to be administered. One of the oral anticancer agents that can be dispersed into water or other fluid, but must be administered immediately after preparation due to lack of stability data, is imatinib. Instructions for parents allege that imatinib is a local irritant and must be taken in a sitting position with a large glass of water (250mL/8oz or at least 100mL/4oz for the children less than 3 years of age) or apple juice instead of water.

In oncology, patients with gastric or head or neck cancer, as well as the elderly belong to another group that is often unable to take medicines in solid oral dosage forms because they have swallowing difficulties or feeding tubes. Conventional and the most common delivery system by ingesting solid forms of drugs is in such cases extremely inconvenient, impractical or even impossible.

Giving imatinib to those patients which have difficulties to swallow tablets or capsules includes steps of placing the required dosage form units into a glass, adding water or apple juice to the glass (approximately 50 mL for a 100 mg dosage form and 200 mL for a 400 mg dosage form), stirring with a spoon until dispersed and giving the suspension immediately to the patient in need. Rinsing the glass with half a glass of water or apple juice for ensured administration of full dose is recommended for extemporaneous oral liquid preparation of imatinib. Orange juice, cola or milk can be not or less appropriate media for imatinib administration, dispersing the tablets in grapefruit juice can be unadmitted or can be less appropriate. Preferably, the fluid for dispersing the pharmaceutical composition comprising imatinib or pharmaceutically acceptable salt thereof can be water or an aqueous liquid different from grapefruit juice, preferably different from grapefruit juice, orange juice, cola and milk.

As shown later in the text by the comparison of disintegration time of composition according to present invention and the marketed conventional tablets of imatinib, the latter are robust and much more slowly disintegrating. The test carried out in accordance with European Pharmacopoeia using purified water at 25 °C as disintegration medium resulted in a disintegration time which was for the reference product up to 20 minutes.

According to NIOSH List of Antineoplastic and Other Hazardous Drugs in Healthcare Settings, 2014, imatinib meets the NIOSH criteria for a hazardous drug. Improperly long, inaccurate and inconvenient preparation, cutting, crushing or otherwise manipulating tablets and capsules to obtain proper liquid oral dosage form presents a potential barrier to adherence and compliance for professionals, patients, family members and caretakers.

Disintegration time might be shortened by opening the capsule of corresponding dosage form of imatinib. Yet another possibility is to apply reconstitution of powder formulation with a diluent just before used as described in WO2013077815. On the other hand, this significantly increases the risk of exposure for the person handling with the drug as well as the possibility of contamination of prepared dosage form and/or work place. Several drawbacks such as dusting, inhalation, spilling out of the content and consequently inaccurate administration, are noticeable.

Ready prepared pharmaceutical solutions are expensive to ship and are bulky for the patient to carry due to the associated mass of the product. Some liquid medicines require fridge storage as chemical, microbiological and physical stability might be impaired.

Approaches to achieve better patient compliance related to the anticancer drug imatinib are known from the prior art. Chewable tablets of imatinib are described in CN101401794(A), in CN101401797(A) and WO2014081172 effervescent tablets containing imatinib mesylate are disclosed and CN101401795(A) relates to orally disintegrating imatinib mesylate tablets. Producing such type of tablets is technologically highly demanding and expensive. They might be characterized by high porosity, low density and/or hardness that make them brittle and difficult to handle. The problem might be also in patients with affected salivary glands and which do not produce enough saliva to smoothly swallow the drug.

A water dispersible tablet formulation of an active pharmaceutical ingredient and one or more adjuvants without use of swellable clay is described in WO2005051350. According to our experience with imatinib dispersible tablet formulations, composition described in WO2005051350 which might according to the inventors be applicable for numerous active pharmaceutical ingredients would not meet the disintegration time within pharmacopoeial requirements, i.e. less than 3 min, since active ingredient loading is too high and disintegrant selection and quantity is not suitable for rapid tablet disintegration. Imatinib is highly cohesive substance exhibiting strong binding properties. This is why high drug loading imatinib tablet would not result in quick disintegration of the tablet, which is even less possible if inadequate quantity and selection of disintegrants are considered.

There is a constant need for development of pharmaceutical formulations, especially anticancer drugs which would be most convenient for patients and would offer advantages over traditional dosage forms. The specific physical and chemical properties of imatinib mesylate and the high doses have been known to cause problems in the preparation of formulations suitable for an oral administration and most relevant recent approaches fail to achieve appropriate therapeutic goals. Considering the prior efforts related to imatinib as disclosed in the background, a need for formulations intended to be dispersed in water or recommended liquid such as apple juice still exists. A homogenous mixture with an appropriate disintegration time, appropriate solubility and dissolution profile ensures an effective oral delivery of active principle being formulated into the pharmaceutical composition according to present invention. In addition, it is desirable to be able to prepare such formulations by using an economical process which avoids the problems of the prior art as noted above.

The object of the present invention is to overcome the drawback of marketed imatinib mesylate oral dosage forms with compositions contributing to better patient compliance. Conventional tablets or capsules of imatinib cannot act as a substitute for a large group of patients. The composition as described herein allows delivering imatinib in a very convenient, safe and efficient manner. Fast disintegrating time, low rate of sedimentation without long lasting handling and waiting for a properly dispersed dose of drug before ready to be administered are characteristics that are particularly interesting for patients who need to be treated with imatinib.

Optimal mechanical strength of final product, drug and dosage form chemical and physical stability, rate of dissolution, absorption and overall bioavailability are additional advantages of the present invention.

Fast dispersible tablets of the present invention constitute a very reliable and efficient way of administering imatinib.

The analysis of the imatinib residue in the glass beaker showed that the whole amount of the active ingredient is administered when the beaker is emptied and that no rinsing of the residue is needed after medicine administration. What is more, the formulation is designed in such a way that the amount of floating particles is reduced to minimum, which can have an important beneficial effect on patient's compliance. In addition, selection of hydrophilic excipients enables good formulation wettability and good dispersion uniformity after mixing the disintegrated preparation. Uniform distribution of the dispersed particles lasts long enough to enable complete active ingredient intake.

### DETAILED DESCRIPTION OF THE INVENTION

The objective of the present invention is to develop new, stable, industrially acceptable, rapidly disintegrating pharmaceutical composition comprising tyrosine-kinase inhibitor, preferably in the form of tablets. Tyrosine kinase-inhibitor, which is imatinib in the form of monomesylate salt, can be used in the pharmaceutical composition of the present invention respecting the therapeutically effective amounts thereof. Selected tyrosine-kinase inhibitor in the present invention is imatinib in the form of monomesylate salt.

The term »imatinib« used as active ingredient in the pharmaceutical composition (which can be a pharmaceutical composition according to present invention or a pharmaceutical composition not according to present invention) refers to above named compound (I) as well as pharmaceutically acceptable salts, enantiomers, racemates, solvates, hydrates, amorphous and crystal forms or combinations thereof. In pharmaceutical composition monomesylate salt of imatinib is used. Most preferably, α or β crystal form of imatinib mesylate is used in the pharmaceutical composition of the present invention. Preparation of α crystal form of imatinib mesylate is disclosed in WO2011157450. It has been found out during the development that particle size of imatinib mesylate have significant impact on processability and final disintegration time of the composition. In case that imatinib mesylate particles are below hereinbelow specified range picking and sticking phenomena can occur during the tableting process due to the increased specific surface. In addition, smaller particles result in stronger inter-particle forces in the composition, which leads to increased disintegration time of the composition. On the other hand, if particle size is too high flowability of the granulate and compression mixture can be harmed, besides homogeneity of the active ingredient in the compression mixture and in the final composition can decrease. Therefore, volume average diameter of imatinib mesylate can be between 2 and 100 µm. More preferably, volume average diameter of imatinib mesylate can be between 3 and 50 µm. Most preferably, volume average diameter of imatinib mesylate can be between 5 and 30 µm in the composition of the present invention.

The formulation may be conducted using standard manufacturing methods and raw materials. According to one aspect of the present invention, there is provided a fast dispersible pharmaceutical composition as defined in claim 1.

According to one aspect of the present invention, there is provided a process for preparation of fast dispersible pharmaceutical composition as defined in claim 12.

According to one aspect of the present invention, there is provided the use of a dispersible pharmaceutical composition in the form of tablet(s) for preparing an oral administration dispersion in water and/or other aqueous liquids before oral administration as defined in claim 14, said dispersible pharmaceutical composition can be especially a fast dispersible pharmaceutical composition in the form of tablet(s),
further in particular a water-dispersible, especially a fast water-dispersible pharmaceutical composition, in the form of tablet(s); in particular, this pharmaceutical composition can be characterized with disintegration time of less than 3 minutes using purified water at 15-25 °C, especially with a disintegration time of less than 3 minutes using purified water at 25 °C. Disintegration time is determined according to European Pharmacopoeia 8.0.

In a pharmaceutical composition of the present invention comprising tyrosine kinase inhibitor, the tyrosine kinase inhibitor is monomesylate salt of imatinib. The amount of imatinib mesylate in the pharmaceutical composition is between 5 and 50 % by weight of the pharmaceutical composition, more preferably between 10 and 40 % by weight, and most preferably between 15 % and 30% by weight of the pharmaceutical composition (based on the total weight of the pharmaceutical composition). In particular, the pharmaceutical composition (which is fast dispersible, in particular fast water-dispersible) is in the form of tablet(s) comprising monomesylate salt of imatinib and further comprising at least two disintegrants; in the combination of at least two disintegrants calcium silicate being one of the disintegrants, a combination of crospovidone and calcium silicate being used. (Said at least two disintegrants can be in particular also referred to herein as a combination of at least two disintegrants.) In particular, for the use of a dispersible pharmaceutical composition (which can be in particular water-dispersible, especially fast dispersible, in particular fast water-dispersible) in the form of tablet(s) for preparing an oral administration dispersion in water and/or other aqueous liquids before oral administration, said dispersible pharmaceutical composition is in the form of tablet(s) comprising monomesylate salt of imatinib and further comprising at least two disintegrants; in the combination of at least two disintegrants calcium silicate being one of the disintegrants, a combination of crospovidone and calcium silicate being used.

A tablet comprising monomesylate salt of imatinib (which is a dispersible, in particular water-dispersible tablet, can be especially fast dispersible, in particular fast water-dispersible tablet) can comprise a mixture, preferably compressed mixture, comprising
(a) granulate, in particular granulate prepared by granulation, in particular wet granulation, said granulate comprising monomesylate salt of imatinib, and
(b) a pharmaceutically acceptable excipient or mixture comprising or consisting of two or more pharmaceutically acceptable excipient(s) (which pharmaceutically acceptable excipient(s) can be also referred to herein as extra-granular excipient(s), and the pharmaceutically acceptable excipient or mixture comprising or consisting of two or more further pharmaceutically acceptable excipient(s) can be also referred to herein as extra-granular phase). According to one embodiment, the terms "granulate" and "granule(s)" can have the same meaning.

The granulate may comprise one or more further pharmaceutically acceptable excipient(s); these one or more further pharmaceutically acceptable excipient(s) can be also referred to herein as intra-granular excipients. Excipient(s) present in the granulate can be also referred to herein as intragranularly used excipient(s). The granulate comprising imatinib or pharmaceutically acceptable salt(s) thereof present in the pharmaceutical composition, especially tablet, can be also referred to herein as intra-granular phase.

The fast dispersible, in particular fast water-dispersible, tablet comprising imatinib monomesylate salt comprises at least two disintegrants; in the combination of at least two disintegrants calcium silicate being one of the disintegrants, a combination of crospovidone and calcium silicate being used. This tablet can be a tablet comprising granulate and an extra-granular phase. In the use of a dispersible pharmaceutical composition in the form of tablet(s) for preparing an oral administration dispersion in water and/or other aqueous liquids before oral administration, a dispersible tablet comprising imatinib monomesylate salt (in particular water-dispersible, especially fast dispersible, in particular fast water-dispersible tablet comprising imatinib monomesylate salt) comprises at least two disintegrants; in the combination of at least two disintegrants calcium silicate being one of the disintegrants, a combination of crospovidone and calcium silicate being used. This tablet can be a tablet comprising granulate and an extra-granular phase. Said extra-granular phase can be a pharmaceutically acceptable excipient or a mixture comprising or consisting of two or more pharmaceutically acceptable excipient(s). The at least two disintegrants (also referred to herein as "combination of at least two disintegrants")
a.) can be present in the extra-granular phase of the tablet, and the granulate comprising imatinib mesylate can be free of said at least two disintegrants, or
b.) can be present both in the extra-granular phase of the tablet, and in the granulate comprising imatinib mesylate, or
c.) a first disintegrant of said at least two disintegrants can be present in the granulate comprising imatinib mesylate, and a second disintegrant (and optionally further disintegrants) of said at least two disintegrants can be present in the extra-granular phase of the tablet, but not present in the granulate comprising imatinib mesylate;
optionally the first disintegrant can be not present in the extra-granular phase. The first disintegrant of said at least two disintegrants can be a disintegrant different from the second disintegrant (and optionally further disintegrants) of said at least two disintegrants.

The pharmaceutical composition comprising monomesylate salt of imatinib (which pharmaceutical composition is a pharmaceutical composition in the form of tablet(s) (which tablet(s) is/are dispersible, in particular water-dispersible tablet(s), can be especially fast dispersible, in particular fast water-dispersible tablet(s)) can comprise:
(i) imatinib mesylate, in a total amount between 5 and 50 % by weight, more preferably between 10 and 40 % by weight, most preferably between 15 % and 30 % by weight, based on the total weight of the pharmaceutical composition,
(ii) disintegrant, preferably in a total amount in the range from 1 % to 50 % by weight, preferably in the range from 5 % to 40 % by weight, most preferably in the range from 8 % to 30 % by weight of the pharmaceutical composition, (said disintegrant being a mixture consisting of two or more disintegrants)
(iii) diluent, preferably in a total amount in the range from 10 % to 80 % by weight, more preferably in the range from 20 % to 70 % by weight, most preferably in the range from 30 % to 60 % by weight of the pharmaceutical composition, (said diluent being a mixture consisting of two or more diluents)
(iv) optionally glidant, preferably in a total amount in the range from 0.01 % to 20 % by weight, more preferably in the range from 0.03 % to 15 % by weight, most preferably in the range from 0.05 % to 10 % by weight, based on the total weight of the pharmaceutical composition, (said glidant can be one glidant or a mixture consisting of two or more glidants)
(v) optionally pH modifier, preferably in a total amount between 0.5 % and 35 % by weight, more preferably between 1.0 and 20 % by weight, most preferably between 2.0 and 15 % by weight of the pharmaceutical composition, (said pH modifier can be one pH modifier or a mixture consisting of two or more pH modifiers)
(vi) lubricant, (said lubricant can be one lubricant or a mixture consisting of two or more lubricants)
(vii) optionally one or more further excipient(s).

A pharmaceutical composition, which is in the form of tablet(s), of the present invention can be obtained e.g. according to any procedure disclosed in the present application, especially by a process comprising the steps:
i) production of imatinib granulate, imatinib being imatinib in the form of monomesylate salt,
ii) blending of imatinib granulate with the extra-granular excipients,
iii) blending a lubricant with a mixture obtained in step ii) to obtain final compression mixture,
iv) compressing of the compression mixture obtained in step iii) into tablets.

A term »fast dispersible tablets« as used herein means tablets intended to be dispersed in water and/or other aqueous liquids such as fruit juices, preferably apple juice, before administration, giving a dispersion, preferably homogeneous dispersion. Dispersion produced passes through a sieve screen with a nominal mesh aperture of 710 µm. Fast dispersible tablets according to present invention disintegrate in less than 3 min using purified water at 25°C. Dispersible tablet of the present invention should be placed in the appropriate volume of beverage, i.e. 100 mL for 400 mg imatinib tablet and 25 mL for 100 mg imatinib tablet, and stirred with a spoon before administration. "Dispersible tablets", "pharmaceutical composition", "pharmaceutical preparation", "pharmaceutical formulation", "composition", "formulation", "medicine", "rapidly disintegrating tablets", "solid medicinal preparation", "final dosage form", "final product", "final composition", "tablets" are all terms used in the present invention which equally characterize "fast dispersible tablets" comprising tyrosine-kinase inhibitor, which is preferably imatinib or pharmaceutically acceptable salts thereof together with at least one pharmaceutically acceptable excipient intended to be dispersed in water and/or other aqueous liquids before oral administration. In particular, a "pharmaceutical composition" (in particular "tablet") of the present invention, e.g. as defined in the claims annexed, represents a "final composition". The indication "% by weight of the final composition" can have in particular the meaning of "% by weight of the pharmaceutical composition, preferably in the form of tablet(s)". Fast dispersible tablets according to present invention can disintegrate in less than 3 min using purified water at 25°C.

Surprisingly, it has been found out that a formulation with fast disintegration, i.e. in less than 3 minutes, comprising tyrosine-kinase inhibitor can be manufactured by using a combination of two or more disintegrants and if these disintegrants are present in the formulation at the predefined ratio.

A combination of disintegrants can be in one embodiment used completely in extra-granular phase or in another embodiment partially in intra-granular and partially in extra-granular phase or in yet another embodiment first disintegrant from the combination can be used entirely or partially intra-granularly and the second and optionally any further disintegrant(s) from the combination can be used entirely in the extra-granular phase of the tablet composition. A combination of at least two disintegrants is used, calcium silicate being one of the disintegrants, a combination of crospovidone and calcium silicate being used. Further disintegrants can be selected from the hereinbelow list of disintegrants. It is also assumed that a selection of disintegrant(s) for intra-granular use should be from the group without hydrogen peroxide or with hydrogen peroxide content. Crospovidone is used as one of the components in the combination of disintegrants. Since hydrogen peroxide can be present in crospovidone as an impurity, adequate chemical stability of the active ingredient is reached especially when the principal amount of this excipient is present in the extra-granular phase. In the case that a small portion of crospovidone is used intra-granularly, a low hydrogen peroxide containing crospovidone grade such as crospovidone having less than 200 ppm of hydrogen peroxide, preferably less than 100 ppm and most preferably less than 50 ppm of hydrogen peroxide is preferential. In case that crospovidone is used as a disintegrant, it is preferred to use types with the volume average diameter of particles within the range of less than 200 µm and more than 10 µm.

Furthermore, the inventors have found out that good processability, rapid tablet disintegration, i.e. in less than three minutes, and adequate appearance of the dispersion after the tablets' disintegration is achieved if hydrophilic tablet lubricant or a combination of hydrophilic and hydrophobic lubricant is incorporated into the formulation.

Moreover, it has been shown that addition of pH-modifier into the formulation shortens disintegration time of the final formulation and increases the solubility of imatinib or pharmaceutically acceptable salt thereof in the formulation.

Last but not least, the applicant has found out that adequate tablet disintegration properties are achieved if formulation is prepared without using a water soluble binder. Formation of the granules with desired physical properties without implementing water soluble binder into the composition can be achieved by performing granulation process using a combination of water and organic solvent as a granulation liquid, where the weight ratio between water and organic solvent is in the range from 3:1 to 1:6, preferably in the range from 2:1 to 1:4 and most preferably in the range from 1.5:1 to 1:3.

It is a principal object of the present invention to provide a manufacturing process which consistently enables manufacturing formulation comprising tyrosine kinase inhibitor, which is imatinib mesylate, in a form of free-flowing and compressible composition, which can be easily compressed into a dispersible tablet. Further, manufactured dispersible tablet has low friability, good compactness, acceptable degree of hardness as well as an adequate dissolution properties and rapid tablet disintegration. After disintegration the dispersion has suitable appearance and is ready to be administered to the patient in need.

In the present invention imatinib is formulated into the granular product with required mechanical properties, such as flowability, compressibility, and compactability by wet granulation process.

Furthermore, the solid medicinal preparation of this invention may comprise various ingredients, according to necessity, such as pharmaceutically acceptable lubricants, glidants, diluents, binders, disintegrants, surfactants, pH modifiers, buffering agents, antioxidant agents, colouring agents, and taste and odour improving agents.

The formulation of the present invention comprises monomesylate salt of imatinib, diluents, one or more glidants, disintegrants, one or more lubricants, and one or more pH modifiers. Optionally, additional excipients may be added to the composition.

It has been found out during the development of the formulation that weight ratio between imatinib or pharmaceutically acceptable salt thereof and final composition has a significant effect on disintegration time. Imatinib or pharmaceutically acceptable salt thereof has cohesive and adhesive properties, thus high drug load of imatinib prolongs disintegration time. Therefore, the amount of imatinib mesylate in the composition of the present invention is between 5 and 50 % by weight of the final composition, more preferably between 10 and 40 % by weight of the final composition, most preferably between 15 % and 30% by weight of the final composition.

Diluents can be selected from starch and its derivatives, such as corn starch, pregelatinized starch, and dextrins, cellulose and its derivatives, such as microcrystalline cellulose or co-processed microcrystalline cellulose such as silicified microcrystalline cellulose, carbohydrates or their derivatives, in particular simple carbohydrates or their derivatives, such as glucose, lactose, and sucrose, sugar alcohols, such as mannitol, xylitol, and sorbitol, metal salts of phosphoric acid, such as calcium hydrogenphosphate in anhydrous or hydrated form, or other diluents and combinations thereof not specifically listed herein. Preferred diluents are mannitol, lactose, sorbitol, microcrystalline cellulose, and silicified microcrystalline cellulose. Particularly preferred diluents are microcrystalline cellulose, silicified microcrystalline cellulose and/or mannitol. A mixture of at least one water insoluble and at least one water soluble diluent is used. Water insoluble and water soluble diluent can be used in weight ratio from 1:10 to 10:1. According to one embodiment, a compound (in particular a diluent) can be especially considered as water soluble when the compound has a solubility of at least 10 g in a liter of water, preferably of at least 25 g in a liter of water (preferably determined at 20 °C, more preferably at 20 °C, 1 atm; the water used for dissolving the compound can have in particular a pH of 7), and a compound (in particular a diluent) can be especially considered as water insoluble when the compound has a solubility of less than 10 g, preferably less than 5 g, more preferably of less than 1 g in a liter of water (preferably at 20 °C, more preferably at 20 °C, 1 atm; the water used for dissolving the compound can have in particular a pH of 7).

A mixture of diluents can be used partially intra- and partially extra-granularly. Preferably, extra-granular portion of microcrystalline cellulose is present in the form of co-processed microcrystalline cellulose. Most preferably, silicified microcrystalline cellulose is used in the extra-granular portion. Preferably, mannitol is present in the form of highly compressible and compactible grade, such as particle engineered types like spray dried and/or granulated mannitol. The combination of diluents according to present invention enables adequate tablet processability and final product characteristics. Preferred amount of diluents in the composition of the present invention is in the range from 10 % to 80 % by weight of the final composition (said final composition can be in particular the pharmaceutical composition of the present invention in the form of tablet(s)), more preferably in the range from 20 % to 70 % by weight of the final composition, most preferably in the range from 30 % to 60 % by weight of the final composition.

Examples of pharmaceutically acceptable glidants that can be used in the composition of the present invention are talc, fumed silica, magnesium oxide, powdered cellulose, silicates, such as magnesium silicate, polyethylene glycols or other glidants or combinations thereof not specifically listed herein. Preferably, silicon dioxide and most preferably colloidal silicon dioxide such as Aerosil^{®} is used in the composition of the present invention. Preferred amount of glidant in the composition of the present invention is in the range from 0.01 % to 20 % by weight of the final composition (said final composition can be in particular the pharmaceutical composition of the present invention in the form of tablet(s)), more preferably in the range from 0.03 % to 15 % by weight of the final composition, most preferably in the range from 0.05 % to 10 % by weight of the final composition.

Disintegrants can be selected from crospovidone, pregelatinized starch, sodium croscarmellose, carmellose sodium or calcium, low substituted hydroxypropyl cellulose, sodium starch glycolate, salts of polacrilin, such as potassium polacrilin, and silicate derivatives, such as calcium silicate or other disintegrants or combinations thereof not specifically listed herein. A combination of crospovidone and calcium silicate is used in the composition of the present invention. It has been found out that the optimal tablet disintegration is achieved when the weight ratio between crospovidone and calcium silicate is between 6:1 and 1:3, preferably between 5:1 and 1:2, and most preferably between 3.5:1 and 1:1.5. Preferred amount of disintegrants in the composition of the present invention is in the range from 1 % to 50 % by weight of the final composition (said final composition can be in particular the pharmaceutical composition of the present invention in the form of tablet(s)), preferably in the range from 5 % to 40 % by weight of the final composition, most preferably in the range from 8 % to 30 % by weight of the final composition.

Chemical stability of imatinib might be harmed in case that an oxidizing agent is present in its microenvironment. Oxidizing agent can lead to the increase of imatinib related substances, such as N-oxide impurity which is listed in Ph. Eur. 8.7 Imatinib mesilate monograph as impurity J (IMP-J). Since crospovidone is a hydrogen peroxide containing material, chemical stability of the formulation might be reduced in its presence. The inventors have found out that adequate chemical stability and rapid tablet disintegration can be achieved in special embodiments of present invention described below. In one embodiment of the present invention, adequate chemical stability of the formulation is achieved by reduced intra-granular crospovidone quantity and using crospovidone with lower amount of hydrogen peroxide. Preferably, hydrogen peroxide content in the crospovidone used in the present invention is not more than 200 ppm, more preferably not more than 100 ppm, and most preferably not more than 50 ppm. Amount of intra-granular crospovidone, which is in intimate contact with drug particles, is preferably less than 50 % of the whole crospovidone quantity, more preferably less than 40 % of the whole crospovidone quantity, most preferably less than 30 % of the whole crospovidone quantity. The weight ratio between imatinib or pharmaceutically acceptable salt thereof and crospovidone present in the granulate of the present invention is preferably more than 3.5:1, more preferably more than 4:1, and even more preferably more than 5:1. In a special embodiment of the present invention two types of crospovidone regarding to their peroxide content can be used in such a way that crospovidone having less than 100 ppm, preferably less than 50 ppm such as but not limited to Polyplasdone^{®} Ultra types produced by company Ashland is used intra-granularly and crospovidone having less than 400 ppm is used extra-granularly.

In another embodiment of the present invention, chemical stability can be achieved by placing complete amount of crospovidone into the extra-granular phase. It was observed that the later solution, i.e. complete amount of crospovidone in the extra-granular phase, results in the highest chemical stability of the formulation.

Calcium silicate is a hydrogen peroxide free excipient, thus it can be used completely in intra-granular phase or in another embodiment partially in intra-granular and partially in extra-granular phase or in yet another embodiment it can be used completely in the extra-granular phase.

Lubricants can be selected from the group consisting of fatty acid, such as stearic acid, a metal salt of fatty acid, such as magnesium stearate, fumed silica, a wax variety, such as cetyl ester waxes or hydrogenated castor oil, boric acid, adipic acid, fumaric acid, sodium stearyl fumarate, sodium lauryl sulphate, magnesium lauryl sulphate, starch derivative, such as corn starch or potato starch, glyceryl behenate, behenoyl polyoxyl glyceride or other lubricants or combinations thereof not specifically listed herein.

In the claimed invention, sodium stearyl fumarate, or sodium stearyl fumarate and magnesium stearate are used as lubricants. Most preferably, combination of sodium stearyl fumarate and magnesium stearate or only sodium stearyl fumarate is used as lubricants. In case that a combination of sodium stearyl fumarate and magnesium stearate is used in the present invention, it is preferred that the weight ratio between sodium stearyl fumarate and magnesium stearate is between 6:1 and 1:4, more preferably 5:1 and 1:3, and even more preferably between 3:1 and 1:2. Most preferably a weight ratio between sodium stearyl fumarate and magnesium stearate is in the weight ratio between 2:1 and 1:1. The applicant has surprisingly found out that such lubrication of the compression mixture enables tableting process without picking and sticking phenomena and at the same time enables rapid tablet disintegration, i.e. in less than 3 minutes using purified water at 15-25 °C, preferably at 25°C.

The optimal disintegration, process, and appearance properties are achieved in case when lubricants are present in the amount between 0.2 % and 15 % by weight of the final composition (said final composition can be in particular the pharmaceutical composition of the present invention in the form of tablet(s)), more preferably in the amount between 0.4 % and 10 % by weight of the final composition, most preferably in the amount between 0.8 % and 5 % by weight of the final composition.

pH modifiers used in the pharmaceutical composition according to the present invention can be selected from the group consisting of organic water soluble mono and/ or polycarboxylic acids such as but not limited to citric acid, malic acid, maleic acid, adipic acid, tartaric, succinic acid, fumaric acid, and/or other pH modifiers or combinations thereof not specifically listed herein. Preferably, citric acid, succinic acid or tartaric acid are used as pH modifiers. More preferably, citric acid is used as a pH modifier. Most preferably anhydrous citric acid is used as a pH modifier.

Imatinib mesylate shows good aqueous solubility at low pH (<5.5) but is poorly soluble or insoluble at neutral and alkaline pH. Addition of pH modifiers to the pharmaceutical composition may contribute to lower pH and consequently better solubility and faster dissolution rate of active ingredient when dispersed in recommended liquid media. It was further found out that addition of pH modifier enables rapid tablet disintegration and leads to better solubility of imatinib when disintegration of the tablet is finished.

The optimal disintegration, solubility, and processability properties are achieved in case when pH modifiers are present in the amount between 0.5 % and 35 % by weight of the final composition, more preferably in the amount between 1.0 % and 20 % by weight of the final composition, most preferably in the amount between 2.0 % and 15 % by weight of the final composition (said final composition can be in particular the pharmaceutical composition of the present invention in the form of tablet(s)).

Optionally, other and further pharmaceutically acceptable excipients such as colorants, surfactants, antioxidant agents, buffering agents, and taste and odour improving agents can be included in the composition.

Colorants that can be used in the pharmaceutical composition according to the present invention and can be selected from the group of pigments such as metal oxides like iron oxides and/or titanium dioxide, and/or from the group of pharmaceutically acceptable water soluble colorants such as indigo carmine or other colorants or combinations thereof not specifically listed herein.

Surfactants that can be used in the pharmaceutical composition according to the present invention can be selected from the group consisting of sodium lauryl sulphate, sorbitan esters, sucrose esters, polysorbates, poloxamers, polyethylene glycol esters, polyoxyethylene alkyl or other surfactants or combinations thereof not specifically listed herein.

In a special embodiment pH of formulation can be optionally adjusted by using buffering agents. Pharmaceutical acceptable buffering agents physically and chemically compatible with the active ingredient and formulation can be used.

Antioxidative agents can be used in the pharmaceutical composition according to the present invention and can be selected from the group consisting of ascorbic acid, sodium ascorbate, ascorbyl palmitate, fumaric acid, malic acid, dodecyl gallate or other antioxidant agents or combinations thereof not specifically listed herein.

Optionally, taste and odour improving agents may be used and can be selected from the group consisting of alginic acid, glyceryl palmitostearate, vanillin, xylitol, fructose, erythritol, polydextrose, cyclodextrin, saccharin, sucralose, menthol, pharmaceutically acceptable flavour mixtures, and other taste and odour improving agents or combinations thereof not specifically listed herein.

The process for the preparation of fast dispersible tablets according to this invention is simple, reliable, straightforward, economical and may be conducted using standard formulation methods. It was found out that optimal process and final composition properties are achieved if firstly imatinib comprising granulate is produced and afterwards extra-granular excipients are admixed to the granulate.

The process of the invention comprises the steps of:
i) production of imatinib granulate, whereby imatinib granulate is a granulate comprising imatinib, imatinib being imatinib in the form of monomesylate salt,
ii) blending of imatinib granulate with the extra-granular excipients,
iii) blending a lubricant with a mixture obtained in step ii) to obtain final compression mixture,
iv) compressing of the compression mixture obtained in step iii) into tablets

Imatinib granulate can in one aspect of the invention comprise imatinib mesylate, one or more diluents, and optionally a glidant. In another aspect of the invention, imatinib containing granulate can additionally comprise a disintegrant having no or low hydrogen peroxide content, imatinib being imatinib in the form of monomesylate salt. In yet another embodiment of the present invention imatinib containing granulate can additionally comprise a pH modifier, imatinib being imatinib in the form of monomesylate salt. However, superior processability and chemical and physical formulation properties are achieved if imatinib granules comprised a drug, a mixture of water soluble and water insoluble diluents, and a glidant, imatinib being imatinib in the form of monomesylate salt. Term imatinib granulate is in present invention used interchangeably with term intra-granular phase.

The amount of imatinib mesylate in the granulate of the present invention is between 5 % and 50 % by weight of the final composition, more preferably between 10 % and 40 % by weight of the final composition, most preferably between 15 % and 30 % by weight of the final composition (said final composition is the pharmaceutical composition of the present invention in the form of tablet(s)). Preferably, mannitol, lactose and/or sorbitol are used as water soluble diluents and microcrystalline cellulose is used as water insoluble diluent in the granulate. More preferably, a mixture of at least one water soluble and at least one water insoluble diluent is used as diluent in the imatinib granulate of the present invention. Most preferably, a mixture of mannitol and microcrystalline cellulose is used as a diluent. Preferred amount of diluents in the granulate of the present invention is between 5 % and 60 % by weight of the final composition, more preferably between 10 % and 50 % by weight of the final composition, most preferably between 18 % and 38 % by weight of the final composition (said final composition is the pharmaceutical composition of the present invention in the form of tablet(s)).

Preferred glidants in the imatinib granulate of the present invention are talc and silicon dioxide. Most preferably, silicon dioxide in the form of colloidal silicon dioxide is used as a glidant in the granulate of the present invention. Preferred amount of glidant in the granulate of the present invention is between 0.01 % and 15 % by weight of the final composition, more preferably between 0.02 % and 10 % by weight of the final composition, most preferably between 0.04 % and 5 % by weight of the final composition (said final composition is the pharmaceutical composition of the present invention in the form of tablet(s)). Optionally, disintegrant without hydrogen peroxide or with low hydrogen peroxide content can be present in the imatinib granulate of the present invention. Preferably, calcium silicate or crospovidone having low hydrogen peroxide content are used in the granulate of the present invention. Disintegrant in the intra-granular phase can be present in the amount up to 15 % by weight of the final composition, more preferably in the amount up to 7.5 % by weight of the final composition (said final composition is the pharmaceutical composition of the present invention in the form of tablet(s)).

Optionally, pH modifier can be present in the imatinib granulate of the present invention. Preferably, citric acid, acetic acid or lactic acid are used as pH modifiers in the granulate. More preferably, citric acid is used as a pH modifier. Most preferably anhydrous citric acid is used as a pH modifier.

It has been found out that optimal tablet disintegration properties are reached if granulation process is performed without using water soluble binders and if the formulation itself is essentially free of water soluble binders. Examples of such unsuitable group of excipients are polyvinylpyrolidone and water soluble cellulose ethers, such as hydroxypropyl methylcellulose, hydroxyethyl cellulose, and hydroxypropyl cellulose.

Wet granulation of tyrosine kinase inhibitor, which is monomesylate salt of imatinib, is performed by the state of the art granulation processes and equipment such as low shear, high shear or fluid bed granulator, where granulation liquid is based on water, one or more acceptable organic solvents or mixture thereof. Preferably, granulation liquid for imatinib granulation is based on a mixture of aqueous and non-aqueous solvents. More preferably, solvent for imatinib wet granulation is a mixture of water and alcohols, such as methanol, ethanol, and isopropanol. Most preferably, granulation liquid is a mixture of water and isopropanol. Preferably, the ratio between water and isopropanol is between 3:1 to 1:6, more preferably between 2:1 and 1:4, most preferably between 1.5:1 and 1:3. It was found out that combination of water and non-aqueous solvent as a granulation liquid enables high wetting of the granulation mixture without too excessive agglomeration which makes the process effective but still feasible. Such process results in physically stable, free-flowing, compressible, and compactible final granules without using the water soluble binder. Moreover, such granules can be compressed in compact dispersible tablets with disintegration time shorter than three minutes.

The applicant has found out that optimal tablet size and process feasibility are attained at the certain bulk volume, tapped volume and particle size distribution of the granulate obtained in step i). Preferred bulk volume of the imatinib granulate is between 1.0 mL/g and 3.5 mL/g, more preferably between 1.5 mL/g and 3.0 mL/g. Preferred tapped volume of the imatinib granulate is between 1.0 and 3.0 mL/g, more preferably between 1.2 mL/g and 2.5 mL/g. Bulk and tapped volume can be determined according to corresponding monographs in Ph. Eur.

Preferably at least 20 %, more preferably at least 30 %, even more preferably at least 35 % of granules produced in step i) are in the particle size range from 71 to 250 µm, determined by sieve analysis. Such particle size distribution of the granulate enables good flow properties and appropriate compression and compaction properties of the compression mixture.

The optimal loss on drying (LOD) value of the imatinib granulate obtained in step i) is in the range from 0.2 % to 2.0 %, preferably from 0.4 % to 1.5 %. Specified LOD enables stable formulation, rapid tablet disintegration and feasibility of the subsequent manufacturing steps. Manufactured imatinib (imatinib being imatinib in the form of monomesylate salt) containing granulate is in the next manufacturing step blended with the extra-granular pharmaceutically acceptable excipients using the blending equipment known from the state of the art such as for example container blender, V-shaped blender, biconical blender, conical blender, horizontal blender, high turbulence blender or high shear mixer.

Preferably, a mixture obtained in step ii) includes one or more diluents, one or more disintegrants, one or more pH modifiers, and one or more glidants. Optionally, other and further pharmaceutically acceptable excipients can be admixed.

Preferably, mannitol, lactose, sorbitol, microcrystalline cellulose, and silicified microcrystalline cellulose can be used as diluents in the extra-granular phase. More preferably, mannitol together with microcrystalline cellulose or silicified microcrystalline cellulose is used. Most preferably, high compressible/compactible grade of mannitol and silicified microcrystalline cellulose are used as diluents in the extra-granular phase. Preferred amount of diluent in the extra-granular phase is between 3 % and 70 % by weight of the final composition, more preferably between 7.5 % and 50% by weight of the final composition, most preferably between 8 % and 38 % by weight of the final composition (said final composition is the pharmaceutical composition of the present invention in the form of tablet(s)).

Preferably, crospovidone and calcium silicate are used as disintegrants in the extra-granular phase. Preferred amount of disintegrant in the extra-granular phase is between 1 % and 45 % by weight of the final composition, more preferably between 3 % and 35 % by weight of the final composition, most preferably between 8 % and 28 % by weight of the final composition (said final composition is the pharmaceutical composition of the present invention in the form of tablet(s)).

Preferably, organic water soluble mono and/ or polycarboxylic acid such as but not limited to citric acid, malic acid, maleic acid, adipic acid, tartaric, succinic acid, fumaric acid, and/or other pH modifiers or combinations thereof not specifically listed herein are used as pH modifiers in the extra-granular phase. More preferably, citric acid is used as a pH modifier. Most preferably anhydrous citric acid is used as a pH modifier. Preferred amount of pH modifier in the extra-granular phase is between 0.5 % and 35 % by weight of the final composition, more preferably between 1.0 and 20 % by weight of the final composition, most preferably between 2.0 and 15 % by weight of the final composition (said final composition is the pharmaceutical composition of the present invention in the form of tablet(s)).

Preferred glidant in the extra-granular phase are talc and silicon dioxide. Most preferably, silicon dioxide in the form of colloidal silicon dioxide is used as glidant in the extra-granular phase. Preferred amount of glidant in the extra-granular phase of the present invention is between 0.01 and 10 % by weight of the final composition, more preferably between 0.02 and 8 % by weight of the final composition, most preferably between 0.05 and 5 % by weight of the final composition (said final composition is the pharmaceutical composition of the present invention in the form of tablet(s)).

Afterwards, one or more lubricants are admixed to the hereinabove described mixture using the state of the art blending equipment.

Preferred lubricants in the extra-granular phase of the present invention are sodium stearyl fumarate, sodium lauryl sulphate, magnesium stearate, glyceryl behenate, and behenoyl polyoxyl-8 gliceride or combinations thereof.

According to the claimed invention, sodium stearyl fumarate or the combination of sodium stearyl fumarate and magnesium stearate are used as lubricants.

The optimal disintegration, process, and dispersion appearance properties are achieved in case when lubricants are present in the extra-granular phase in the amount between 0.2 % and 15 % by weight of the final composition, more preferably in the amount between 0.4 % and 10 % by weight of the final composition, most preferably in the amount between 0.8 % and 5 % by weight of the final composition (said final composition can be in particular the pharmaceutical composition of the present invention in the form of tablet(s)).

After the lubricant admixing, compression mixture is compressed into tablets. The applicant has found out that such lubrication of the compression mixture enables tableting process without picking and sticking phenomena. The applicant has found out that optimal tablet size and process feasibility are attained at the certain bulk volume, tapped volume and particle size distribution. Preferred bulk volume of the imatinib compression mixture is between 1.0 mL/g and 3.5 mL/g, more preferably between 1.4 mL/g and 2.8 mL/g. Preferred tapped volume of the imatinib compression mixture is between 1.0 and 3.0 mL/g, more preferably between 1.1 mL/g and 2.3 mL/g.

The optimal loss on drying (LOD) value of the final compression mixture obtained in point iii) is in the range from 0.2 to 3.0 %, preferably from 0.5 % to 2.5 %. Specified LOD enables stable formulation, rapid tablet disintegration and feasibility of the further manufacturing process.

Preferably at least 20 %, more preferably at least 25 %, even more preferably at least 30 % of the compression mixture is in the particle size range from 71 to 250 µm. Such particle size distribution of compression mixture enables good flow properties and appropriate compression and compaction properties of the compression mixture.

Final stage of the dispersible tablet manufacturing is compressing the compression mixture using the state of the art tableting equipment. The physical characteristics of the tablets are suitable for packaging on a conventional packaging line. The tablets of the present invention correspond to all pharmacopoeial standards for tablets and dispersible tablets. The tablets enable good wettability and dispersion uniformity after mixing with the recommended disintegration liquid, the tablets are dispersed in less than 3 minutes and ensures complete active ingredient intake.

The applicant has also found out that the disintegration time below 3 min is possible if tablet hardness does not exceed certain value. Hardness of the tablet is less than 160 N, preferably less than 130 N, and even more preferably less than 100 N.

Tablet hardness was measured using Erweka Multicheck machine, every time 20 tablets were tested and average tablet hardness was calculated.

Further, the invention relates to proper packaging of the final dosage form that is chosen in such a way that oxygen and other environmental effects are minimized.

The pharmaceutical formulation of the present invention can be packaged in accordance with procedures and materials known from the state of the art. For example, as packaging material blisters, glass bottles, containers made of polymeric materials with suitable closure systems may be used. Packaging can be performed under normal atmosphere, under an atmosphere having a reduced oxygen concentration and, preferably, in an inert atmosphere. Packaging under reduced relative humidity, preferably below 40% relative humidity, can also be performed. Reduced oxygen concentration means that the concentration of oxygen in the gas surrounding the solid composition in the primary packaging is below 18 % V/V, preferably below 10 % V/V and most preferably below 5 % V/V.

According to a preferred embodiment, contact between the pharmaceutical composition and environmental oxygen may be reduced or suppressed by either packaging the pharmaceutical composition under reduced pressure, packaging in an inert gas atmosphere, or by using a packaging wherein the contact between the pharmaceutical composition and oxygen is reduced by the means of oxygen absorbers.

An atmosphere with reduced oxygen content or reduced oxygen partial pressure may be obtained by the use of an atmosphere of reduced pressure, e.g. by creating a partial vacuum by means of a suitable pump or by partial freezing or liquefying the atmosphere, by the use of an inert gas atmosphere, wherein as an inert gas nitrogen or argon may be used, or by the use of absorbents. Suitable absorbents may be selected from the group of commercially available absorbents such as humidity-activated oxygen absorbers, ultravioletradiation-activated absorbers, radiation-activated absorbers, microwaves-radiation-activated absorbers, absorbers activated by a combination of activation processes or absorbers without necessity of activation. Examples of commercially available absorbers are Ageless^{™} (Mitsubishi Gas Chemical), ATCO (Standa Industry), FreshPax^{™} (Multisorb Technologies), O-Buster^{™} (Hsiao Sung Non-Oxygen Chemical Co), Biotika Oxygen Absorber (Biotika) and the like.

The invention also provides a stabilized package which is provided with a space for trapping and disposal of free oxygen. Moreover, if the active compounds of the present composition are exhibited to a reduced oxygen partial pressure, the formulation is preferably enclosed in a substantially gas exchange non-permeable material and an atmosphere with reduced oxygen partial pressure is contained in the packaging. The substantially gas exchange non-permeable package is preferably selected from the group consisting of an Al/Al blister, an Al-polychloro-3-fluoroethylene homopolymer/PVC laminate blister.

Final packaging can optionally contain dessicant which can be used in blisters made of aluminium foil, incorporated into closure system of glass and/or polymeric containers or added directly into containers.

Preferably, the objective of the present invention is packed in Al/Al blisters or Al-polychloro-3-fluoroethylene homopolymer/PVC laminate blister packed under normal or inert gas atmosphere. More preferably, the objective of the present invention is packed in Al/Al blisters under normal or inert gas atmosphere. Most preferably, the objective of the present invention is packed in Al/Al blisters under the inert gas atmosphere.

The present invention can be illustrated in one of its embodiments by the following nonlimiting examples.

All quantities of raw materials in composition for each example are given in amounts for one tablet.

### ANALYTICAL METHODS

Bulk volume was measured using Method 1 (Measurement in a Graduated Cylinder) as prescribed in European Pharmacopoeia 8.0, chapter 2.9.34. Bulk Density and Tapped Density of Powders, paragraph Bulk density. Bulk volume was calculated by dividing measured volume with the weight of the applied material.

The average particle size of imatinib mesylate was determined by laser diffraction method using Malvern Mastersizer MS2000 equipped with Hydro 2000S dispersion unit. Isopar L was used as dispersion medium. A small homogeneous sample representative to the population of particles was collected and adequately dispersed. For the sample preparation 1% solution of epikuron 100SP1 in Isopar L was used in order to adequately wet the particles. The term average particle size as used herein refers to the volume mean particle diameter.

Particle size measurements of both granulates and compression mixture were conducted using air jet sieve particle size analyzer (Hosokawa Alpine 200 LS). Sieves with mesh size 71, 125, 250, 500 and 710 µm were used to carry out the analysis.

Tapped density was measured using Method 1 as prescribed in European Pharmacopoeia 8.0, chapter 2.9.34. Bulk Density and Tapped Density of Powders, paragraph Tapped density. Volume was read after 1250 taps had been applied. Tapped volume was calculated by dividing measured volume with the weight of the applied material.

LOD measurements were done on a 5 g sample using Mettler Toledo halogen moisture analyzer set at 105 °C for 5 min.

Disintegration testing was carried out in apparatus A or B (European Pharmacopoeia) using purified water at the temperature of interest (in particular a room temperature) as disintegration medium. The test was carried out in accordance with European Pharmacopoeia 8.0, chapter 2.9.1.

For determining disintegration time of a pharmaceutical composition of less than 3 minutes using purified water at 15-25 °C (in particular 25°C), the test can be carried out in accordance with European Pharmacopoeia 8.0, especially European Pharmacopoeia 8.0, chapter 2.9.1, using purified water at 15-25 °C (in particular 25°C) for disintegrating the pharmaceutical composition, applying in particular apparatus A or B (apparatus A or B can be chosen according to the instructions provided in European Pharmacopoeia 8.0, especially European Pharmacopoeia 8.0, chapter 2.9.1; discs being used in the apparatus used according to the instructions provided in European Pharmacopoeia 8.0, especially European Pharmacopoeia 8.0, chapter 2.9.1). Purified water can be purified water (Aqua purificata) in accordance with European Pharmacopoeia 8.0, especially purified water having a pH of 7.

### EXAMPLES 1-5

### (Examples 1, 2, 4, 5 are not falling within the subject-matter for which protection is sought)

| **Material** | **Ex. 1** | **Ex. 2** | **Ex. 3** | **Ex. 4** | **Ex. 5** |
|---|---|---|---|---|---|
| **Granulate** (mg) | | | | | |
| **Imatinib Mesylate** | 478 | 478 | 478 | 478 | 478 |
| **Mannitol** | 265 | 265 | 250 | 255 | 260 |
| **Microcrystalline Cellulose** | 130 | 130 | 159 | / | 130 |
| **Calcium Silicate** | / | / | / | 140 | / |
| **Crospovidone** | 40 | 40 | / | 40 | / |
| **Low Substituted Hydroxypropyl Cellulose** | / | / | / | / | 40 |
| **Fumed Silica (Colloidal Silicon Dioxide)** | 4 | 4 | 4 | 4 | 4 |
| **Isopropanol** | q.s. | q.s. | q.s. | q.s. | q.s. |
| **Purified Water** | q.s. | q.s. | q.s. | q.s. | q.s. |

| **Compression mixture** (mg) | | | | | |
|---|---|---|---|---|---|
| **Mannitol** | 200 | 165 | / | 165 | 150 |
| **Calcium Silicate** | 165 | 200 | 120 | 200 | 115 |
| **Microcrystalline Cellulose** | / | / | 197 | / | 100 |
| **Crospovidone** | 140 | 140 | 180 | 140 | / |
| **Low Substituted Hydroxypropyl Cellulose** | / | / | / | / | 140 |
| **Fumed Silica (Colloidal Silicon Dioxide)** | 4 | 4 | 4 | 4 | 4 |
| **Magnesium Stearate** | 14 | 14 | / | 14 | 14 |
| **Sodium Stearyl Fumarate** | / | / | 45 | / | / |
| **Tablet weight** | 1440 | 1440 | 1440 | 1440 | 1440 |

| | | | | | |
|---|---|---|---|---|---|
| *Granulation liquid was prepared by mixing Isopropanol and Purified water in the ratio 2:1. | | | | | |

Imatinib mesylate, intra-granular portion of diluents, disintegrants, and glidants were homogenized in a granulating machine. Afterwards, granulation liquid comprising isopropanol and purified water was sprayed onto the powder mixture. The obtained granulate was dried in the fluid bed drier until the product temperature reached temperature between 35 °C and 45 °C.

Extra-granular components were admixed to the imatinib granulate to obtain final compression mixture.

Finally, compression mixture was compressed into a tablet.

### EXAMPLE 6

Disintegration testing was carried out on Examples 1, 4, and 5 in apparatus A (European Pharmacopoeia) using purified water at 25 °C as disintegration medium. The test was carried out in accordance with European Pharmacopoeia.

| **Example** | **Ex. 1** | **Ex. 4** | **Ex. 5** | **Reference Gleevec 400 mg, Batch No. S0026** |
|---|---|---|---|---|
| **Disintegration time** | 3 min 31 s | 4 min 2 s | 8 min 33 s | 17 min 13 s |

It was observed that Ex. 5, tablet comprising no crospovidone, had a very long disintegration time. Examples 1, 4, 5 are not falling within the subject-matter for which protection is sought.

### EXAMPLES 7-16 (Examples 7, 8, 12 are not falling within the subject-matter for which protection is sought)

| **Material** | **Ex. 7** | **Ex. 8** | **Ex. 9** | **Ex. 10** | **Ex. 11** |
|---|---|---|---|---|---|
| **Granulate** (mg) | | | | | |
| **Imatinib Mesylate** | 478 | 478 | 478 | 478 | 478 |
| **Mannitol** | 262 | 262 | 240 | 262 | 540 |
| **Microcrystalline Cellulose** | 270 | 270 | 280 | 270 | |
| **Crospovidone (Kollidon^{®} CL)** | 70 | 70 | / | 70 | 40 |
| **Crospovidone (Polyplasdone^{™} Ultra)** | / | / | / | / | / |
| **Fumed Silica (Colloidal Silicon Dioxide)** | 4 | 4 | 4 | 4.00 | / |
| **Isopropanol*** | q.s. | q.s. | q.s. | q.s. | q.s. |
| **Purified Water*** | q.s. | q.s. | q.s. | q.s. | q.s. |

| **Compression mixture** (mg) | | | | | |
|---|---|---|---|---|---|
| **Mannitol** | 250 | 230 | 230 | 210 | / |
| **Calcium Silicate** | 120 | 120 | 120 | 120 | 100 |
| **Silicified Microcrystalline Cellulose** | 200 | 200 | / | 200 | / |
| **Microcrystalline Cellulose** | / | / | 200 | / | 422 |
| **Crospovidone (Kollidon CL)** | 200 | 200 | 254 | 200 | 220 |
| **Fumed Silica (Colloidal Silicon Dioxide)** | 6 | 6 | 12 | 6 | 10 |
| **Magnesium Stearate** | 20 | 40 | / | / | / |
| **Sodium Stearyl Fumarate** | / | / | 50 | 60 | 40 |
| **Behenoyl Polyoxyl-8 Glyceride** | / | / | 12 | / | 30 |
| **Tablet weight** | 1880 | 1880 | 1880 | 1880 | 1880 |

| | | | | | |
|---|---|---|---|---|---|
| *Granulation liquid was prepared by mixing Isopropanol and Purified water in the ratio 2:1. | | | | | |

| | **Ex. 12** | **Ex. 13** | **Ex. 14** | **Ex. 15** | **Ex. 16** |
|---|---|---|---|---|---|
| **Granulate (mg)** | | | | | |
| **Imatinib Mesylate** | 478 | 478 | 478 | 478 | 478 |
| **Mannitol** | 262 | 262 | 262 | / | 262 |
| **Microcrystalline Cellulose** | 270 | 270 | 270 | 520 | 270 |
| **Crospovidone (Kollidon^{®} CL)** | 70 | / | / | / | / |
| **Crospovidone (Polyplasdone ^{™} Ultra)** | / | / | 70 | / | / |
| **Calcium Silicate** | / | 70 | / | 70 | / |
| **Fumed Silica (Colloidal Silicon Dioxide)** | 4 | 4 | 4 | / | 4 |
| **Isopropanol*** | q.s. | q.s. | q.s. | q.s. | q.s. |
| **Purified Water*** | q.s. | q.s. | q.s. | q.s. | q.s. |

| **Compression mixture** (mg) | | | | | |
|---|---|---|---|---|---|
| **Mannitol** | 175 | 210 | 210 | 200 | 210 |
| **Calcium Silicate** | 120 | 120 | 120 | 150 | 120 |
| **Silicified Microcrystalline Cellulose** | 200 | / | 200 | / | 200 |
| **Microcrystalline Cellulose** | / | 200 | / | 194 | / |
| **Crospovidone (Kollidon^{®} CL)** | 200 | / | 200 | 200 | 270 |
| **Crospovidone(Polyplasdone ^{™} Ultra)** | / | 200 | / | / | / |
| **Fumed Silica (Colloidal Silicon Dioxide)** | 6 | 6 | 6 | 6 | 6 |
| **Magnesium Stearate** | / | / | / | 10 | / |
| **Sodium Stearyl Fumarate** | / | 60 | 60 | 40 | 60 |
| **Behenoyl Polyoxyl-8 Glyceride** | 95 | / | / | 12 | / |
| **Tablet weight** | 1880 | 1880 | 1880 | 1880 | 1880 |

| | | | | | |
|---|---|---|---|---|---|
| *Granulation liquid was prepared by mixing Isopropanol and Purified water in the ratio 2:1. | | | | | |

Imatinib mesylate and intra-granular excipients were homogenized in a granulating machine. Afterwards, granulation liquid comprising isopropanol and purified water was sprayed onto the homogenized powder mixture. The obtained granulate was dried in the fluid bed drier until the product temperature reached temperature between 35 °C and 45 °C.

Extra-granular components were admixed to the imatinib granulate to obtain final compression mixture.

Finally, compression mixture was compressed into a tablet.

### EXAMPLE 17

Disintegration time testing was carried out on Examples 8, 10, and 12 in apparatus B (European Pharmacopoeia) using purified water at 25 °C as disintegration medium. The test was carried out in accordance with European Pharmacopoeia.

| **Example** | **Ex. 8** | **Ex. 10** | **Ex. 12** | **Reference Gleevec 400 mg, Batch No. S0026** |
|---|---|---|---|---|
| **Disintegration time** | 2 min 26 s | 2 min 11 s | 2 min 58 s | 17 min 13 s |

It was observed that Ex. 10, comprising only sodium stearyl fumarate as lubricant, had the fastest tablet disintegration. All of the formulations met the Ph. Eur. requirements for dispersible tablets (not more than 3 min), however, Ex. 12 had disintegration time very close to the specified limit.

### EXAMPLE 18

Stability results of example 10 and 14 were compared.

Samples produced as described hereinabove were stored for 1 month at 50 °C/75 % RH in closed air-tight packaging. Increase in concentration of impurity IMP-J, which is main imatinib chemical impurity, was investigated.

| **Storage conditions** | **Increase of IMP-J Ex. 10** | **Increase of IMP-J Ex. 14** |
|---|---|---|
| **t₀** | 0.04 | 0.01 |
| **50 °C/ 75 % RH 1 month** | 0.21 | 0.09 |

It was observed that chemical stability of Ex. 14 was significantly better than chemical stability of Ex. 10.

### EXAMPLE 19

Stability results of Ex. 14 and 16 were compared.

Samples produced as described hereinabove were stored for 1 month at 50 °C/75 % RH in closed air-tight packaging. Increase in concentration of impurity IMP-J, which is main imatinib chemical impurity, was investigated.

| **Storage conditions** | **Increase of IMP-J Ex. 14** | **Increase of IMP-J Ex. 16** |
|---|---|---|
| **t₀** | 0.01 | 0.01 |
| **50 °C/ 75 % RH 1 month** | 0.09 | 0.06 |

It was observed that chemical stability of Ex. 16 was better than stability of Ex. 14.

### EXAMPLES 20-25 (Examples 21 and 22 are not falling within the subject-matter for which protection is sought)

| | **Ex. 20** | **Ex. 21** | **Ex. 22** | **Ex. 23** | **Ex. 24** | **Ex. 25** |
|---|---|---|---|---|---|---|
| **Granulate** (mg) | | | | | | |
| **Imatinib Mesylate** | 478 | 478 | 478 | 478 | 478 | 478 |
| **Mannitol (Parteck^{®} M 200)** | 332 | 262 | 340 | 262 | 262 | 262 |
| **Microcrystalline Cellulose PH-102** | 200 | 270 | 192 | 270 | 270 | 270 |
| **Crospovidone (Polyplasdone ^{™} Ultra)** | 70 | / | / | / | / | / |
| **Fumed Silica (Colloidal Silicon Dioxide)** | 4 | 4 | 4 | 4 | 4 | 4 |
| **Isopropanol*** | q.s. | q.s. | q.s. | q.s. | q.s. | q.s. |
| **Purified Water*** | q.s. | q.s. | q.s. | q.s. | q.s. | q.s. |

| **Compression mixture** (mg) | | | | | | |
|---|---|---|---|---|---|---|
| **Mannitol (Parteck^{®} M 200)** | 150 | 170 | 170 | 160 | 160 | 210 |
| **Calcium Silicate (RxCIPIENTS^{®} FM1000)** | 120 | 120 | 120 | 120 | 120 | 120 |
| **Silicified Microcrystalline Cellulose (Prosolv^{®} SMCC 90)** | 160 | 160 | 160 | 160 | 150 | 200 |
| **Crospovidone (Kollidon^{®} CL)** | 200 | 270 | 270 | 270 | 270 | 270 |
| **Fumed Silica (Colloidal Silicon Dioxide)** | 6 | 6 | 6 | 6 | 6 | 6 |
| **Citric acid, anhydrous** | 100 | 100 | 100 | 100 | 100 | / |
| **Sodium stearyl fumarate** | 60 | / | / | 30 | 60 | 60 |
| **Magnesium stearate** | / | 40 | 40 | 20 | / | / |
| **Tablet weight** | 1880 | 1880 | 1880 | 1880 | 1880 | 1880 |

| | | | | | | |
|---|---|---|---|---|---|---|
| * Granulation liquid was prepared by mixing Isopropanol and Purified water in the ratio 2:1. | | | | | | |

Imatinib mesylate and intra-granular excipients were homogenized in a granulating machine. Afterwards, granulation liquid comprising isopropanol and purified water was sprayed onto the homogenized powder mixture. The obtained granulate was dried in the fluid bed drier until the product temperature reached temperature between 35 °C and 45 °C.

Extra-granular components were admixed to the imatinib granulate to obtain final compression mixture.

Finally, compression mixture was compressed into a tablet. Weight of the final 400 mg dispersible tablet is 1880 mg. The compression mixture can be also used for the production of 100 mg dispersible tablets with a tablet weight of 470 mg.

### EXAMPLES 26

Disintegration time testing was carried out on Examples 24 and 25 in apparatus B (European Pharmacopoeia) using water at 25 °C as disintegration medium. The test was carried out in accordance with European Pharmacopoeia.

| **Example** | **Ex. 24** | **Ex. 25** | **Reference Gleevec 400 mg, Batch No. S0026** |
|---|---|---|---|
| **Disintegration time** | 24 s | 1 min 4 s | 17 min 13 s |

It was obvious that incorporation of citric acid into the formulation Ex. 24 significantly improved tablet disintegration time.

### EXAMPLE 27

| | **Ex. 27** |
|---|---|
| **Granulate** (mg) | |
| **Imatinib Mesylate** | 478 |
| **Mannitol** | 262 |
| **Microcrystalline Cellulose** | 270 |
| **Fumed Silica (Colloidal Silicon Dioxide)** | 4 |
| **Isopropanol*** | q.s. |
| **Purified Water*** | q.s. |

| **Compression mixture** (mg) | |
|---|---|
| **Mannitol** | 157 |
| **Calcium Silicate** | 120 |
| **Silicified Microcrystalline Cellulose** | 150 |
| **Crospovidone** | 270 |
| **Fumed Silica (Colloidal Silicon Dioxide)** | 6 |
| **Sucralose** | 1.5 |
| **Banana flavour**** | 1.5 |
| **Citric acid, anhydrous** | 100 |
| **Sodium stearyl fumarate** | 60 |
| **Tablet weight** | 1880 |

| | |
|---|---|
| *Granulation liquid was prepared by mixing Isopropanol and Purified water in the ratio 2: 1. **In the form of concentrated mixture of flavours. | |

Imatinib mesylate and intra-granular excipients were homogenized in a granulating machine. Afterwards, granulation liquid comprising isopropanol and purified water was sprayed onto the homogenized powder mixture. The obtained granulate was dried in the fluid bed drier until the product temperature reached temperature between 35 °C and 45 °C.

Extra-granular components were admixed to the imatinib granulate to obtain final compression mixture.

Finally, compression mixture was compressed into a tablet.

## Claims

1. Fast dispersible pharmaceutical composition in the form of tablet(s), said fast dispersible pharmaceutical composition comprising tyrosine-kinase inhibitor with at least one pharmaceutically acceptable excipient intended to be dispersed in water and/or other aqueous liquids before oral administration,
wherein said tyrosine kinase inhibitor is monomesylate salt of imatinib,
wherein a combination of lubricants sodium stearyl fumarate and magnesium stearate or sodium stearyl fumarate as lubricant is used, and
wherein a combination of at least two disintegrants is used,
said fast dispersible pharmaceutical composition being characterized with disintegration time of less than 3 minutes using purified water at 25°C, the disintegration test being carried out in accordance with European Pharmacopoeia 8.0, and
**characterized in that** in the combination of at least two disintegrants calcium silicate is one of the disintegrants, a combination of crospovidone and calcium silicate being used, and **in that** the proportion of active ingredient is between 5 and 50 % by weight of the final composition, and **in that**
a mixture of at least one water insoluble and at least one water soluble diluent is used.

2. Fast dispersible tablet(s) of monomesylate salt of imatinib according to claim 1, wherein the proportion of active ingredient is between 10 and 40 % by weight of the final composition, and most preferably between 15 % and 30% by weight of the final composition.

3. Fast dispersible tablet(s) of monomesylate salt of imatinib according to any one of claims 1 to 2, wherein a combination of at least two disintegrants is used completely in extra-granular phase or partially in intra-granular and partially in extra-granular phase or first disintegrant from the combination can be used entirely or partially intra-granularly and the second and optionally any further disintegrant(s) from the combination can be used entirely in the extra-granular phase of the tablet composition.

4. Fast dispersible tablet(s) of monomesylate salt of imatinib according to any of claims 1 to 3, wherein a combination of crospovidone and calcium silicate is used, in the weight ratio between 6:1 and 1:3, preferably between 5:1 and 1:2, and most preferably between 3.5:1 and 1:1.5.

5. Fast dispersible tablet(s) of monomesylate salt of imatinib according to any one of claims 1 to 4, wherein the principle amount of crospovidone is present in the extra-granular phase or in case that a small portion of crospovidone is used intra-granularly, a low hydrogen peroxide containing crospovidone grade such as crospovidone having less than 200 ppm of hydrogen peroxide, preferably less than 100 ppm and most preferably less than 50 ppm of hydrogen peroxide is used.

6. Fast dispersible tablet(s) of monomesylate salt of imatinib according to any of claims 1 to 5, wherein pH modifier(s) are selected from the group consisting of organic water soluble mono and/ or polycarboxylic acids such as citric acid, malic acid, maleic acid, adipic acid, tartaric, succinic acid, fumaric acid, and/or other pH modifiers or combinations thereof, and wherein preferably said citric acid, succinic acid or tartaric acid, more preferably citric acid, and most preferably anhydrous citric acid as pH modifier is used.

7. Fast dispersible tablet(s) of monomesylate salt of imatinib according to any of claims 1 to 6, wherein diluents are selected from starch and its derivatives, such as corn starch, pregelatinized starch, and dextrins, cellulose and its derivatives, such as microcrystalline cellulose or co-processed microcrystalline cellulose such as silicified microcrystalline cellulose, simple carbohydrates or their derivatives, such as glucose, lactose, and sucrose, sugar alcohols, such as mannitol, xylitol, and sorbitol, metal salts of phosphoric acid, such as calcium hydrogenphosphate in anhydrous or hydrated form, and combinations thereof, and wherein preferably microcrystalline cellulose, silicified microcrystalline cellulose and/or mannitol as diluents are used.

8. Fast dispersible tablet(s) of monomesylate salt of imatinib according to any of claims 1 to 7, wherein a mixture of at least one water insoluble and at least one water soluble diluent in a weight ratio from 1:10 to 10:1 is used.

9. Fast dispersible tablet(s) of monomesylate salt of imatinib according to any of claims 1 to 8, wherein glidant(s) are selected from talc, fumed silica, magnesium oxide, powdered cellulose, magnesium silicate, polyethylene glycols or combinations thereof, wherein preferably silicon dioxide and most preferably colloidal silicon dioxide as glidant is used.

10. Fast dispersible tablet(s) of monomesylate salt of imatinib according to any of claims 1 to 9, wherein a combination of lubricants sodium stearyl fumarate and magnesium stearate is used, in the weight ratio between 6:1 and 1:4, preferably between 5:1 and 1:3, more preferably between 3:1 and 1:2, and most preferably between 2:1 and 1:1.

11. Fast dispersible tablet(s) of monomesylate salt of imatinib according to any of claims 1 to 10, wherein pharmaceutical composition is prepared without using a water soluble binder.

12. The process for preparation of fast dispersible pharmaceutical composition comprising monomesylate salt of imatinib according to any one of claims 1 to 11, wherein the process comprises the steps of:
i) production of imatinib granulate, imatinib being imatinib in the form of monomesylate salt,
ii) blending of imatinib granulate with the extra-granular excipients, imatinib being imatinib in the form of monomesylate salt,
iii) blending a lubricant with a mixture obtained in step ii) to obtain final compression mixture,
iv) compressing of the compression mixture obtained in step iii) into tablets.

13. The process according to claim 12, wherein said process is wet granulation and where granulation liquid is based on water, one or more acceptable organic solvents or mixture thereof.

14. Use of a dispersible pharmaceutical composition in the form of tablet(s) for preparing an oral administration dispersion in water and/or other aqueous liquids before oral administration,
said dispersible pharmaceutical composition comprising tyrosine-kinase inhibitor with at least one pharmaceutically acceptable excipient, said tyrosine kinase inhibitor being monomesylate salt of imatinib, and
said dispersible pharmaceutical composition being a dispersible pharmaceutical composition wherein the proportion of active ingredient is between 5 and 50 % by weight of the final composition, and
said dispersible pharmaceutical composition being a dispersible pharmaceutical composition wherein a combination of lubricants sodium stearyl fumarate and magnesium stearate or sodium stearyl fumarate as lubricant is used, and
wherein a combination of at least two disintegrants is used, in the combination of at least two disintegrants calcium silicate being one of the disintegrants, a combination of crospovidone and calcium silicate being used, and wherein a mixture of at least one water insoluble and at least one water soluble diluent is used.

15. Use of a dispersible pharmaceutical composition in the form of tablet(s) for preparing an oral administration dispersion in water and/or other aqueous liquids before oral administration according to claim 14, **characterized in that** the dispersible pharmaceutical composition is a fast dispersible composition according to any one of claims 1 to 11.

## Patentansprüche

1. Schnell dispergierbare pharmazeutische Zusammensetzung in der Form von Tablette(n), wobei die schnell dispergierbare pharmazeutische Zusammensetzung Tyrosin-Kinase-Inhibitor mit mindestens einem pharmazeutisch verträglichen Exzipienten umfasst, vorgesehen zum Dispergieren in Wasser und/oder anderen wässrigen Flüssigkeiten vor oraler Verabreichung,
wobei der Tyrosin-Kinase-Inhibitor Monomesylat-Salz von Imatinib ist,
wobei eine Kombination von Gleitmitteln Natriumstearylfumarat und Magnesiumstearat oder Natriumstearylfumarat als Gleitmittel verwendet wird, und wobei eine Kombination von mindestens zwei Sprengmitteln verwendet wird,
wobei die schnell dispergierbare pharmazeutische Zusammensetzung durch eine Zerfallszeit von kürzer als 3 Minuten unter Verwendung von gereinigtem Wasser bei 25°C gekennzeichnet ist, wobei der Zerfallstest gemäß dem Europäischen Arzneibuch 8.0 durchgeführt wird, und
**dadurch gekennzeichnet, dass** in der Kombination von mindestens zwei Sprengmitteln Calciumsilicat eines der Sprengmittel ist, eine Kombination von Crospovidon und Calciumsilicat verwendet wird, und dass der Anteil an Wirkstoff zwischen 5 und 50 Gew.-% der finalen Zusammensetzung liegt, und dass
ein Gemisch von mindestens einem wasserunlöslichen und mindestens einem wasserlöslichen Verdünnungsmittel verwendet wird.

2. Schnell dispergierbare Tablette(n) von Monomesylat-Salz von Imatinib gemäß Anspruch 1, wobei der Anteil an Wirkstoff zwischen 10 und 40 Gew.-% der finalen Zusammensetzung, und am stärksten bevorzugt zwischen 15 Gew.-% und 30 Gew.-% der finalen Zusammensetzung liegt.

3. Schnell dispergierbare Tablette(n) von Monomesylat-Salz von Imatinib gemäß einem der Ansprüche 1 bis 2, wobei eine Kombination von mindestens zwei Sprengmitteln vollständig in extra-granulärer Phase oder teilweise in intra-granulärer und teilweise in extra-granulärer Phase verwendet wird, oder erstes Sprengmittel von der Kombination gänzlich oder teilweise intra-granulär verwendet werden kann und das zweite und gegebenenfalls jedwede(s) weitere Sprengmittel von der Kombination gänzlich in der extra-granulären Phase der Tablettenzusammensetzung verwendet werden kann/können.

4. Schnell dispergierbare Tablette(n) von Monomesylat-Salz von Imatinib gemäß einem der Ansprüche 1 bis 3, wobei eine Kombination von Crospovidon und Calciumsilicat verwendet wird, im Gewichtsverhältnis zwischen 6:1 und 1:3, bevorzugt zwischen 5:1 und 1:2, und am stärksten bevorzugt zwischen 3,5:1 und 1:1,5.

5. Schnell dispergierbare Tablette(n) von Monomesylat-Salz von Imatinib gemäß einem der Ansprüche 1 bis 4, wobei die Grundbestandteilmenge an Crospovidon in der extra-granulären Phase vorhanden ist, oder im Falle, dass ein kleiner Teil von Crospovidon intra-granulär verwendet wird, eine Qualität von Crospovidon mit niedrigem Wasserstoffperoxid-Gehalt wie Crospovidon mit weniger als 200 ppm Wasserstoffperoxid, bevorzugt weniger als 100 ppm und am stärksten bevorzugt weniger als 50 ppm Wasserstoffperoxid verwendet wird.

6. Schnell dispergierbare Tablette(n) von Monomesylat-Salz von Imatinib gemäß einem der Ansprüche 1 bis 5, wobei pH-Wert-verändernde(s) Mittel aus der Gruppe ausgewählt ist/sind, welche aus organischen wasserlöslichen Mono- und/oder Polycarbonsäuren wie Zitronensäure, Äpfelsäure, Maleinsäure, Adipinsäure, Weinsäure, Bernsteinsäure, Fumarsäure, und/oder anderen pH-Wert-verändernden Mitteln oder Kombinationen davon besteht, und wobei bevorzugt die Zitronensäure, Bernsteinsäure oder Weinsäure, stärker bevorzugt Zitronensäure, und am stärksten bevorzugt wasserfreie Zitronensäure als pH-Wert-veränderndes Mittel verwendet wird.

7. Schnell dispergierbare Tablette(n) von Monomesylat-Salz von Imatinib gemäß einem der Ansprüche 1 bis 6, wobei Verdünnungsmittel aus Stärke und ihren Derivaten, wie Maisstärke, prägelierter Stärke, und Dextrinen, Cellulose und ihren Derivaten, wie mikrokristalliner Cellulose oder co-verarbeiteter mikrokristalliner Cellulose wie verkieselter mikrokristalliner Cellulose, einfachen Kohlenhydraten oder ihren Derivaten, wie Glucose, Lactose, und Saccharose, Zuckeralkoholen, wie Mannitol, Xylitol, und Sorbitol, Metallsalzen von Phosphorsäure, wie Calciumhydrogenphosphat in wasserfreier oder hydratisierter Form, und Kombinationen davon, ausgewählt sind, und wobei bevorzugt mikrokristalline Cellulose, verkieselte mikrokristalline Cellulose und/oder Mannitol als Verdünnungsmittel verwendet werden.

8. Schnell dispergierbare Tablette(n) von Monomesylat-Salz von Imatinib gemäß einem der Ansprüche 1 bis 7, wobei ein Gemisch von mindestens einem wasserunlöslichen und mindestens einem wasserlöslichen Verdünnungsmittel in einem Gewichtsverhältnis von 1:10 bis 10:1 verwendet wird.

9. Schnell dispergierbare Tablette(n) von Monomesylat-Salz von Imatinib gemäß einem der Ansprüche 1 bis 8, wobei Fließreguliermittel aus Talk, Quarzstaub, Magnesiumoxid, Cellulosepulver, Magnesiumsilicat, Polyethylenglykolen oder Kombinationen davon ausgewählt ist/sind, wobei bevorzugt Siliciumdioxid und am stärksten bevorzugt kolloidales Siliciumdioxid als Fließreguliermittel verwendet wird.

10. Schnell dispergierbare Tablette(n) von Monomesylat-Salz von Imatinib gemäß einem der Ansprüche 1 bis 9, wobei eine Kombination von Gleitmitteln Natriumstearylfumarat und Magnesiumstearat verwendet wird, im Gewichtsverhältnis zwischen 6:1 und 1:4, bevorzugt zwischen 5:1 und 1:3, stärker bevorzugt zwischen 3:1 und 1:2, und am stärksten bevorzugt zwischen 2:1 und 1:1.

11. Schnell dispergierbare Tablette(n) von Monomesylat-Salz von Imatinib gemäß einem der Ansprüche 1 bis 10, wobei eine pharmazeutische Zusammensetzung ohne Verwendung eines wasserlöslichen Bindemittels hergestellt wird.

12. Verfahren zur Herstellung einer schnell dispergierbaren pharmazeutischen Zusammensetzung umfassend Monomesylat-Salz von Imatinib gemäß einem der Ansprüche 1 bis 11, wobei das Verfahren die folgenden Schritte umfasst:
i) Herstellung von Imatinib-Granulat, wobei Imatinib Imatinib in der Form von Monomesylat-Salz ist,
ii) Mischen von Imatinib-Granulat mit den extra-granulären Exzipienten, wobei Imatinib Imatinib in der Form von Monomesylat-Salz ist,
iii) Mischen eines Gleitmittels mit einem in Schritt ii) erhaltenen Gemisch, wobei finales Verpressungsgemisch erhalten wird,
iv) Verpressen des in Schritt iii) erhaltenen Verpressungsgemisches zu Tabletten.

13. Verfahren gemäß Anspruch 12, wobei das Verfahren Nassgranulierung ist und wobei Granulierungsflüssigkeit auf Wasser, einem oder mehr verträglichen organischen Lösungsmitteln oder Gemisch davon basiert.

14. Verwendung einer dispergierbaren pharmazeutischen Zusammensetzung in der Form von Tablette(n) zum Herstellen einer orale Verabreichung-Dispersion in Wasser und/oder anderen wässrigen Flüssigkeiten vor oraler Verabreichung,
wobei die dispergierbare pharmazeutische Zusammensetzung Tyrosin-Kinase-Inhibitor mit mindestens einem pharmazeutisch verträglichen Exzipienten umfasst, wobei der Tyrosin-Kinase-Inhibitor Monomesylat-Salz von Imatinib ist, und
wobei die dispergierbare pharmazeutische Zusammensetzung eine dispergierbare pharmazeutische Zusammensetzung, wobei der Anteil an Wirkstoff zwischen 5 und 50 Gew.-% der finalen Zusammensetzung liegt, ist, und
wobei die dispergierbare pharmazeutische Zusammensetzung eine dispergierbare pharmazeutische Zusammensetzung, wobei eine Kombination von Gleitmitteln Natriumstearylfumarat und Magnesiumstearat oder Natriumstearylfumarat als Gleitmittel verwendet wird, ist, und
wobei eine Kombination von mindestens zwei Sprengmitteln verwendet wird, in der Kombination von mindestens zwei Sprengmitteln Calciumsilicat eines von den Sprengmitteln ist, eine Kombination von Crospovidon und Calciumsilicat verwendet wird, und wobei ein Gemisch von mindestens einem wasserunlöslichen und mindestens einem wasserlöslichen Verdünnungsmittel verwendet wird.

15. Verwendung einer dispergierbaren pharmazeutischen Zusammensetzung in der Form von Tablette(n) zum Herstellen einer orale Verabreichung-Dispersion in Wasser und/oder anderen wässrigen Flüssigkeiten vor oraler Verabreichung gemäß Anspruch 14, **dadurch gekennzeichnet, dass** die dispergierbare pharmazeutische Zusammensetzung eine schnell dispergierbare Zusammensetzung gemäß einem der Ansprüche 1 bis 11 ist.

## Revendications

1. Composition pharmaceutique à dispersion rapide sous forme de comprimé(s), ladite composition pharmaceutique à dispersion rapide comprenant un inhibiteur de la tyrosine kinase avec au moins un excipient pharmaceutiquement acceptable, destinée à être dispersée dans l'eau et/ou d'autres liquides aqueux avant l'administration par voie orale,
dans lequel ledit inhibiteur de la tyrosine kinase est un sel de monomésylate d'imatinib,
dans lequel une combinaison de lubrifiants, le stéarylfumarate de sodium et le stéarate de magnésium ou le stéarylfumarate de sodium, est utilisée comme lubrifiant, et
dans lequel une combinaison d'au moins deux désintégrants est utilisée,
ladite composition pharmaceutique à dispersion rapide étant **caractérisée par** un temps de désintégration inférieur à 3 minutes en utilisant de l'eau purifiée à 25°C, le test de désintégration étant effectué conformément à la Pharmacopée Européenne 8.0, et
**caractérisée en ce que** dans la combinaison d'au moins deux désintégrants, le silicate de calcium est l'un des désintégrants, une combinaison de crospovidone et de silicate de calcium étant utilisée, et **en ce que** la proportion de principe actif est comprise entre 5 et 50% en poids de la composition finale, et **en ce que**
un mélange d'au moins un diluant insoluble dans l'eau et d'au moins un diluant soluble dans l'eau est utilisé.

2. Comprimé(s) à dispersion rapide de sel de monomésylate d'imatinib selon la revendication 1, dans lequel la proportion de principe actif est comprise entre 10 et 40% en poids de la composition finale, et de préférence entre 15% et 30% en poids de la composition finale.

3. Comprimé(s) à dispersion rapide de sel de monomésylate d'imatinib selon l'une quelconque des revendications 1 à 2, dans lequel une combinaison d'au moins deux désintégrants est utilisée complètement en phase extra-granulaire ou partiellement en phase intra-granulaire et partiellement en phase extra-granulaire, ou le premier désintégrant de la combinaison peut être utilisé entièrement ou partiellement de manière intra-granulaire et le second et éventuellement tout autre désintégrant de la combinaison peuvent être utilisés entièrement dans la phase extra-granulaire de la composition en comprimés.

4. Comprimé(s) à dispersion rapide de sel de monomésylate d'imatinib selon l'une quelconque des revendications 1 à 3, dans lequel une combinaison de crospovidone et de silicate de calcium est utilisée, dans un rapport pondéral compris entre 6:1 et 1:3, de préférence entre 5:1 et 1:2, et plus préférablement entre 3,5:1 et 1:1,5.

5. Comprimé(s) à dispersion rapide de sel de monomésylate d'imatinib selon l'une quelconque des revendications 1 à 4, dans lequel la quantité de principe de crospovidone est présente dans la phase extra-granulaire, ou dans le cas où une petite partie de crospovidone est utilisée de manière intra-granulaire, une qualité de crospovidone à faible teneur en peroxyde d'hydrogène, telle que la crospovidone, ayant moins de 200 ppm de peroxyde d'hydrogène, de préférence moins de 100 ppm et plus préférablement moins de 50 ppm de peroxyde d'hydrogène, est utilisée.

6. Comprimé(s) à dispersion rapide de sel de monomésylate d'imatinib selon l'une quelconque des revendications 1 à 5, dans lequel le(s) modificateur(s) de pH est(sont) choisi(s) dans le groupe composé d'acides monocarboxyliques et/ou polycarboxyliques organiques solubles dans l'eau tels que l'acide citrique, l'acide malique, l'acide maléique, l'acide adipique, l'acide tartrique, l'acide succinique, l'acide fumarique, et/ou d'autres modificateurs de pH ou leurs combinaisons, et dans lesquels, de préférence, ledit acide citrique, acide succinique ou acide tartrique, plus préférablement l'acide citrique, et encore plus préférablement, l'acide citrique anhydre est utilisé comme modificateur de pH.

7. Comprimé(s) à dispersion rapide de sel de monomésylate d'imatinib selon l'une quelconque des revendications 1 à 6, dans lequel les diluants sont choisis parmi l'amidon et ses dérivés, tels que l'amidon de maïs, l'amidon prégélatinisé et les dextrines, la cellulose et ses dérivés, tels que la cellulose microcristalline ou la cellulose microcristalline cotraitée, telle que la cellulose microcristalline silicifiée, les glucides simples ou leurs dérivés, tels que le glucose, le lactose et le saccharose, les alcools de sucre, tels que le mannitol, le xylitol et le sorbitol, les sels métalliques de l'acide phosphorique, tels que le phosphate d'hydrogène de calcium sous forme anhydre ou hydratée, et leurs combinaisons, et dans lequel de préférence la cellulose microcristalline, la cellulose microcristalline silicifiée et/ou le mannitol sont utilisés comme diluants.

8. Comprimé(s) à dispersion rapide de sel de monomésylate d'imatinib selon l'une quelconque des revendications 1 à 7, dans lequel un mélange d'au moins un diluant insoluble dans l'eau et d'au moins un diluant soluble dans l'eau, dans un rapport pondéral de 1:10 à 10:1, est utilisé.

9. Comprimé(s) à dispersion rapide de sel de monomésylate d'imatinib selon l'une quelconque des revendications 1 à 8, dans lequel le(s) agent(s) glissant(s) est(sont) choisi(s) parmi le talc, la silice pyrogénée, l'oxyde de magnésium, la cellulose en poudre, le silicate de magnésium, les polyéthylèneglycols ou leurs combinaisons, dans lequel de préférence le dioxyde de silicium, et encore plus préférablement le dioxyde de silicium colloïdal est utilisé comme glissant.

10. Comprimé(s) à dispersion rapide de sel de monomésylate d'imatinib selon l'une quelconque des revendications 1 à 9, dans lequel une combinaison de lubrifiants, le stéarylfumarate de sodium et le stéarate de magnésium, est utilisée, dans le rapport pondéral compris entre 6:1 et 1:4, de préférence entre 5:1 et 1:3, plus préférablement entre 3:1 et 1:2, et encore plus préférablement entre 2:1 et 1:1.

11. Comprimé(s) à dispersion rapide de sel de monomésylate d'imatinib selon l'une quelconque des revendications 1 à 10, dans lequel la composition pharmaceutique est préparée sans utiliser de liant soluble dans l'eau.

12. Procédé de préparation d'une composition pharmaceutique à dispersion rapide comprenant le sel de monomésylate d'imatinib selon l'une quelconque des revendications 1 à 11, dans lequel le procédé comprend les étapes de :
i) production de granulat d'imatinib, l'imatinib étant l'imatinib sous forme de sel de monomésylate,
ii) mélange du granulat d'imatinib avec les excipients extragranulaires, l'imatinib étant l'imatinib sous forme de sel de monomésylate,
iii) mélange d'un lubrifiant avec un mélange obtenu à l'étape ii) pour obtenir le mélange de compression final,
iv) compression du mélange de compression obtenu à l'étape iii) en comprimés.

13. Procédé selon la revendication 12, dans lequel ledit procédé est une granulation par voie humide et dans lequel le liquide de granulation est à base d'eau, d'un ou plusieurs solvants organiques acceptables ou de leur mélange.

14. Utilisation d'une composition pharmaceutique dispersible sous forme de comprimés (s) pour préparer une dispersion par administration orale dans l'eau et/ou d'autres liquides aqueux avant administration orale,
ladite composition pharmaceutique dispersible comprenant un inhibiteur de la tyrosine kinase avec au moins un excipient pharmaceutiquement acceptable, ledit inhibiteur de la tyrosine kinase étant le sel de monomésylate d'imatinib, et
ladite composition pharmaceutique dispersible étant une composition pharmaceutique dispersible dans laquelle la proportion de principe actif est comprise entre 5 et 50% en poids de la composition finale, et
ladite composition pharmaceutique dispersible étant une composition pharmaceutique dispersible dans laquelle une combinaison de lubrifiants, le stéarylfumarate de sodium et le stéarate de magnésium ou le stéarylfumarate de sodium, est utilisée comme lubrifiant, et
dans laquelle une combinaison d'au moins deux désintégrants est utilisée, dans la combinaison d'au moins deux désintégrants, le silicate de calcium étant l'un des désintégrants, une combinaison de crospovidone et de silicate de calcium étant utilisée, et dans lequel un mélange d'au moins un diluant insoluble dans l'eau et d'au moins un diluant soluble dans l'eau est également utilisé.

15. Utilisation d'une composition pharmaceutique dispersible sous forme de comprimé(s) pour préparer une dispersion pour administration orale dans l'eau et/ou d'autres liquides aqueux avant administration orale selon la revendication 14, **caractérisée en ce que** la composition pharmaceutique dispersible est une composition à dispersion rapide selon l'une quelconque des revendications 1 à 11.
